# EUROPEAN PATENT SPECIFICATION

(11) **EP 1 487 591 B1**
(45) Date of publication and mention of the grant of the patent: **22.03.2006**
(21) Application number: 03712397.3
(22) Date of filing: 24.03.2003
(51) Int. Cl.: B05D 7/24, G01N 33/543

(54) **PREPARATION OF COATINGS THROUGH PLASMA POLYMERIZATION**
HERSTELLUNG VON BESCHICHTUNGEN DURCH PLASMAPOLYMERISATION
PREPARATION DE REVETEMENTS AU MOYEN DE LA POLYMERISATION PAR PLASMA

(30) Priority: 28.03.2002 GB 0207350
(43) Date of publication of application: 22.12.2004
(73) Proprietor: Plasso Technology Limited, Sheffield S1 4DP (GB)
(72) Inventor: Short, Rob, Sheffield S1 4DT (GB); Whittle, Jason, Sheffield S10 2TN (GB); Shard, Alex G., Sheffield S10 2TN (GB); Barton, David, Sheffield S10 2TN (GB)
(74) Representative: Boakes, Jason Carrington
(86) International application number: PCT/GB2003/001242
(87) International publication number: WO 2003/082483

(56) References cited:
- WO-A-01/31339
- WO-A-02/32591
- US-A- 5 545 531
- US-A- 5 976 466
- US-B1- 6 306 506

## Description

The invention relates to a method to manufacture a non-uniform plasma polymerised surface and products comprising a surface obtainable by said method.

Molecular architecture is the formation of three-dimensional structures of polymeric material on surfaces that have controllable levels of crosslinking, frictional wear or solubility characteristics. Chemical architecture refers to the engineering of chemical functionality (the presence of certain reactive moieties, or groups). These surfaces may have utility in assay products, mass spectrometer probes, microfludic systems, or in microarray devices, or in micromachines as valves, switches, or pumps.

Currently the use of solid phase assay systems has greatly facilitated the processing and/or analysis of multiple biological samples. This has become a highly automated methodology. Typically, solid phase assays comprise either the immobilisation of the agent to be assayed on a solid, or at least semi-solid, surface or the immobilisation of agents used to assay a biological agent. The results derived from such assays have greatly assisted clinicians in their diagnosis of various human disorders. They have also enabled environmental authorities to monitor the presence of environmental pollutants and the presence of various infectious agents that may be present in our environment and/or food. Assays of this type are often laborious and time consuming. It is important that assays are sensitive and reliable.

Genomics analysis involves the analysis of sequence information (DNA, RNA or protein) typically generated from genome sequencing projects. Typically biomolecules immobilised for this purpose are referred to as microarrays. An array is a two-dimensional sheet to which is applied different biomolecules at different sites on the sheet. This facilitates the screening of the biomolecules in parallel and on a much smaller scale than conventional solid phase assays. Typically biomolecules are immobilised by chemical coupling or adsorption. Currently arrays of biomolecules are made by depositing aliquots of sample under conditions which allow the molecules to bind or be bound to the array surface. Alternatively, or in addition, biomolecules maybe synthesised at the array surface and directly or indirectly immobilised. The number of different samples that are applied to a single array can reach thousands. The application of samples to form an array can be facilitated by the use of "array printers", (for example see Gene Expression Micro-Arrays, A New Tool for Genomics, Shalon, D, in Functional Genomics, IBC library series; Southern EM, DNA Chips: Analysing Sequence by Hybridisation to Oligonucleotides on a Large Scale, Trends in Genetics, 12: 110-5, 1996). The analysis of micro-arrays is undertaken by commercially available "array readers" which are used to interpolate the data generated from the array, for example as disclosed in US5, 545, 531. Arrays are typically made individually and used only once before being disposed of. Therefore, it is highly desirable to produce arrays which are manufactured to a high degree of reproducibility and with minimum error.

Similarly the recent genomics projects have generated a substantial amount of protein sequence information. This has greatly facilitated structure/function analysis of proteins to assist in the assigning of function to novel protein sequences. Typically this sort of analysis is referred to as proteomics.

Microarray substrates are typically manufactured from glass, plastics (e.g. polyethylene terephthalate, high density polyethylene, low density polyethylene, polyvinyl chloride, polypropylene or polystyrene); nitrocellulose, nylon.

Typically, solid phase assays are conducted in assay dishes containing multiple wells that are coated with the molecule of interest. These multi-well application dishes are normally manufactured either from glass or plastics that may have variable affinity for the molecule(s) of interest. Plastics used in the manufacture of assay products include polyethylene terephthalate, high density polyethylene, low density polyethylene, polyvinyl chloride, polypropylene or polystyrene.

Multi-well dishes can be treated chemically to improve their affinity and/or retention of selected molecules at their surface. It is, of course, highly desirable that the treated surface binds with the target molecule with high affinity and retention but also allows the bound molecule to retain most, if not all, of its biological activity thereby providing a sensitive and reliable assay.

An example of such a treatment regime for solid phase surfaces is described in GB2016687. The patent describes the treatment of binding surfaces with polysaccharides. Surfaces treated in this way show increased affinity for both antibodies and antigens. WO8603840 describes solid phase assay surfaces manufactured from specialised resins as an alternative to the use of assay containers manufactured from plastics such as polystyrene. Specifically, W08603840 discloses the use of the fluorinated resin polytetrafluoroethylene. WO9819161 describes the coating of solid phase assay surfaces with polyethyleneimine. The treated surfaces show low levels of non-specific adsorption and a high concentration of binding of the target molecule.

Microfluidic systems are scaled-down fluid flow devices, in which the dimensions of the device are such that the surface tension forces dominate that of gravity. As a result of this, the properties of the internal surfaces of the device have a massive influence on the efficacy of the device. Typically a microfluidic device is constructed from a polymer, such as polycarbonate, or from silicon.

Also, a "lab on a chip" is a scaled down laboratory experiment, or series of experiments which allows conventional techniques to be applied on a small scale.

In WO01/31339 we disclose the treatment of products by plasma polymerisation.

Plasma polymerisation is a technique which allows an ultra-thin (eg ca.200nm) cross linked polymeric film to be deposited on substrates of complex geometry and with controllable chemical functionality. As a consequence, the surface chemistry of materials can be modified, without affecting the bulk properties of the substrate so treated. Plasmas or ionised gases are commonly excited by means of an electric field. They are highly reactive chemical environments comprising ions, electrons, neutrals (radicals, metastables, ground and excited state species) and electromagnetic radiation. At reduced pressure, a regime may be achieved where the temperature of the electrons differs substantially from that of the ions and neutrals. Such plasmas are referred to as "cold" or "non-equilibrium" plasmas. In such an environment many volatile organic compounds (eg volatile alcohol containing compounds, volatile acid containing compounds, volatile amine containing compounds, or volatile hydrocarbons, neat or with other gases, eg Ar, have been shown to polymerise (H.K. Yasuda, Plasma Polymerisation, Academic Press, London 1985) coating both surfaces in contact with the plasma and those downstream of the discharge. The organic compound is often referred to as the "monomer". The deposit is often referred to as "plasma polymer". The advantages of such a mode of polymerisation potentially include: ultra-thin pin-hole free film deposition; plasma polymers can be deposited onto a wide range of substrates; the process is solvent free and the plasma polymer is free of contamination. Under conditions of low power, typically 10⁻² W/cm³, plasma polymer films can be prepared which retain a substantial degree of the chemistry of the original monomer. For example, plasma polymerised films of acrylic acid contain the carboxyl group (Haddow et al., Langmuir, Vol 16: 5654-60, 2000). The low power regime may be achieved either by lowering the continuous wave power, or by pulsing the power on and off.

Co-polymerisation of one or more compounds having functional groups with a hydrocarbon allows a degree of control over surface functional group concentrations in the resultant plasma copolymer (PCP) (Beck et al., Polymer 37: 5537-5539, 1996). Suitably, the monomers are ethylenically unsaturated. Thus the functional group compound maybe unsaturated carboxylic acid, alcohol or amine, for example, whilst the hydrocarbon is suitably an alkene. By plasma polymerisation, it is also possible to deposit ethylene oxide-type molecules (eg. tetraethyleneglycol monoallyl ether) to form 'non-fouling' surfaces (Beyer et al., Journal of Biomedical Materials Research 36: 181-9, 1997). It is also possible to deposit perfluoro-compounds (i.e. perfluorohexane, hexafluoropropylene oxide) to form hydrophobic/superhydrophobic surfaces (Coulson et al., Chemistry of Materials 12: 2031-2038, 2000).

This technique is advantageous because the surfaces have unique chemical and physical characteristics. For example, the surfaces have increased affinity for biological molecules exposed to said surface and allow the assaying of the bound molecule. The surfaces are uniform and enable the reproducible and sensitive assaying of biological molecules bound to the surface. Similarly, the surface wettability, adhesion and frictional/wear characteristics of the substrate can be modified in a controllable and predictable manner.

The technique disclosed in WO01/31339, although effective with respect to providing uniform plasma polymerised surfaces to which biomolecules bind with specificity and affinity, is not sufficiently versatile to provide a surface which has diverse chemical or physical properties.

The method herein disclosed allows the provision of surfaces that are non-uniform and define local surface regions that have different chemical and/or physical properties. We refer to these surfaces as "patterned" in both chemistry and topography. The effect is achieved by drawing off a proportion of the plasma through a micrometre scale orifice(s) which is translated across the surfaces to be patterned. Alternatively, a plasma may be excited at the tip, or within a microcapilliary which can then be used to "write" the molecular architecture and chemistry onto the surface. Chemistry and molecular architecture maybe varied vertically (Z-direction) and/or laterally (X-Y plane) by changing the key plasma parameters (power, flow rate, pulse duty cycle or monomer composition), or by altering the portion of the plasma 'drawn off by physical, electrical or magnetic means during writing. These surfaces allow the immobilisation of different molecules and concentrations of molecules at a micron scale. Similarly, this technique may be used to control the local wettability, adhesion and frictional/wear characteristics on a surface, and have application in microfluidics.

The combination of chemistry and topography permits the fabrication of micrometre scale structures that can act as switches, valves and pumps.

We herein disclose a method we refer to as "plasma writing" which provides surfaces that are characterised by chemical and structural micropattems or gradients extending, typically into three dimensions, wherein the X-Y plane is defined by the surface, and the Z-direction is substantially perpendicular thereto. The invention relates to a method of creating both chemical and molecular architectures onto a surface, to give rise to two or three-dimensional patterns, without the need to prefabricate masks or stencils, as described in Dai et al., Journal of Physical Chemistry B 101:9548-54 (1997) and without limitation in the number or type of different architectures created on a single surface as part of the same process.

According to an aspect of the invention there is provided a method to deposit a non-uniform plasma polymerised surface to a substrate.

Non-uniform refers to surfaces which have a heterogeneous chemical and/or physical structure.

According to a further aspect of the invention there is provided a method to prepare at least part of at least one surface of a substrate comprising; depositing on said surface at least one plasma monomer wherein during deposition of said monomer, means are provided which move the monomer source across a surface to be treated to manufacture a non-uniform polymer surface.

In a yet further aspect there is provided a method to prepare at least part of at least one surface of a substrate comprising: depositing on said substrate surface at least one plasma monomer wherein during deposition of said monomer, means are provided which cause relative movement of the monomer source and the substrate surface to be treated to manufacture a non-uniform plasma polymer surface.

In a preferred method of the invention said means moves said substrate relative to said monomer source.

In an alternative method of the invention said means moves said monomer source relative to said substrate.

The substrate and plasma source are affixed to either side of a precision XYZ translation stage. The XYZ stage comprises one fixed and one travelling flange. Therefore, the substrate and plasma source are moved relative to each other.

The invention herein disclosed enables the deposition of plasma polymers with different chemistries and molecular architecture in a spatially restricted pattern, optionally at varying concentration, and at a micrometer resolution. This allows the production of products with highly defined chemical and physical surface properties which advantageously; facilitates the binding and/or separation of different biological molecules and different concentrations of biological molecules followed by their detection and analysis; locally modifies the surface characteristics such as wettability, friction and wear, and adhesion; and fabricates structures which through a combination of chemistry and structure act as switches, valves or pumps (upon receipt of an appropriate stimulus).

In a preferred method of the invention there is provided a surface comprising two or more plasma polymers formed from at least two monomers, preferably a plurality of plasma polymers formed from a plurality of monomers.

In a further preferred method of the invention said surface comprises at least one plasma polymer of at least one monomer wherein the concentration of said plasma polymer is non-uniform across said surface, or part thereof.

In a further preferred method of the invention, said surface comprises of two or more plasma polymers of two or more monomers, wherein the concentration of at least one plasma polymer is non-uniform across said surface, or part thereof.

In a further preferred method of the invention said monomer is a volatile alcohol.

In an alternative method of the invention said monomer pattern is a volatile acid.

In a still further alternative method said monomer is a volatile amine.

In a further method of the invention said monomer is a volatile hydrocarbon.

In a yet further preferred method of the invention said monomer is a volatile fluorocarbon.

In a still further preferred method of the invention said monomer is an ethyleneoxide-type molecule.

In a further preferred method of the invention said monomer is a volatile siloxane.

In yet still a further preferred method of the invention said monomer is at least one of selected from the group consisting of: allyl alcohol; acrylic acid; octa-1,7-diene; allyl amine; perfluorohexane; tetraethyleneglycol monoallyl ether; or hexamethyl disiloxane (HMDSO).

In a further preferred method of the invention said polymer consists of a single monomer.

Preferably the monomer consists essentially of an ethylenically unsaturated organic compound.

Preferably the monomer consists of essentially of a single ethylenically unsaturated organic compound.

Preferably the monomer consists of an ethylene oxide type molecule. (e.g. Triglyme)

Preferably the compound is an alkene (eg containing up to 20 carbon atoms and more usually up to 12 carbon atoms, eg 8), a carboxylic acid (especially α,β - unsaturated carboxylic acid, for example acrylic or methacrylic acid); an alcohol (especially an unsaturated alcohol); or an amine (especially an unsaturated amine).

Preferably the monomer consists of a mixture of two or more ethylenically unsaturated organic compounds.

Preferably the compounds are selected from the group consisting of: an alkene (eg containing up to 20 carbon atoms and more usually up to 12 carbon atoms, eg 8), a carboxylic acid (especially α,β - unsaturated carboxylic acid); an alcohol (especially an unsaturated alcohol); or an amine (especially an unsaturated amine).

"Alkene" refers to linear and branched alkenes, of which linear are preferred, containing one or more than one C=C double bond eg an octadiene such as octa-1,7-diene. Dienes form a preferred class of alkenes.

The monomer may consist essentially of a saturated organic compound. The monomer may consist of an aromatic compound, a heterocyclic compound or a compound containing one or more carbon-carbon triple bonds.

Alternatively said polymer is a co-polymer. Preferably said co-polymer comprises at least one organic monomer with at least one hydrocarbon. Preferably said hydrocarbon is an alkene, eg a diene such as, for example octa 1,7-diene.

The method also encompasses the use of other compounds to form plasma, for example and not by way of limitation, ethylamine; heptylamine; methacrylic acid; propanol, hexane, acetylene or diaminopropane.

Preferably the monomer is a polymerisable monomer having a vapour pressure of at least 6.6x10⁻² mbar. Monomers with a vapour pressure of less than 1.3x10⁻² mbar are generally not suitable unless their vapour pressure can be raised sufficiently by heating.

In a preferred method of the invention said monomer (s) is/are deposited on said surface in spatially separated dots.

In a further preferred method of the invention said monomer (s) is/are deposited on said surface in tracks or lines.

In a yet further preferred method of the invention, said dots and/or lines may be of different polymer chemistry.

In a still further preferred method of the invention, the chemical composition and/or functionality of the line, track or dot may be non-uniform along its length and in height.

In a yet further preferred method of the invention, the line or track may be in the form of loops or closed circuits.

In a yet further preferred method of the invention regions which do not consist of a deposited plasma polymer may be comprised of polymerised ethylene-oxide type monomer providing a non-binding surface.

In a preferred method of the invention said plasma is sustained under low power conditions, from which are obtainable films containing the original monomer chemistry. Typically, low power conditions refer to a continuous wave power of 10⁻² W/cm³, or the equivalent time-averaged power in the case of pulsed plasmas.

According to a further aspect of the invention there is provided a substrate comprising a surface obtainable by the method according to the invention.

Preferably said substrate is selected from the group consisting of: glass; plastics (e.g. polyethylene terephthalate, high density polyethylene, low density polyethylene, polyvinyl chloride, polypropylene or polystyrene); nitrocellulose, or nylon, metal, ceramics, quartz, composite structures (e.g. metal fihn on glass) or silicon wafer.

In a preferred embodiment of the invention said substrate is part of an assay product.

In a further preferred embodiment of the invention said assay product is a microarray.

In an alternative preferred embodiment said assay product is microtitre plate.

In an alternative preferred embodiment said product is a probe component for use in a mass spectrometer.

In a further embodiment said surface is part of a product for separating cells and/or proteins and/or macromolecules. The surface may be part of an affinity purification matrix.

In an alternative preferred embodiment said substrate comprises a microfluidic device, or a part thereof (e.g. valve, switch, guide channel, binding site, pump).

It will be apparent that the invention relates to the provision of plasma polymerised surfaces that are physically non-uniform and which we refer to as "patterned". The invention relates to the provision of surfaces of more than one single patterned chemistry (indeed, there is no practical limit on the number) that can be 'drawn' with micrometre precision. Such surfaces cannot be obtained by the stencil approach of Dai et al.(Journal of Physical Chemistry B 101: 9548-54, 1997). The morphology of the substrate merely affects the maximum resolution of the plasma pattern.

Patterns may consist of lines, circles, loops, arrays, or any conceivable geometric shape in any combination on a scale from centimetres down to around 5 microns. This includes 3-dimensional patterns where the material along the z-axis (height) also exhibits chemical and physical differences. As such, nanometer features may be 'grown' on surfaces which comprise different strata of "chemistry" on different local regions of the surface.

A polymer may be deposited from virtually any compound (particularly organic compounds), provided it can be induced to form a plasma. Typically this means that the compound must be volatile, although this may be done by heating or by use of a carrier gas, for example monomers having a vapour pressure of at least 6.6x10⁻² mbar at room temperature. Hence a microdot or array thereof, or a microtrack may be produced which contains any chemical functional group and there is no limit to the number of different chemistries that may be deposited onto a single substrate. This is in contrast to previously disclosed methods of patterning plasma polymers, which are only capable of depositing 'monotone' patterns. The written polymer does not necessarily contain any functional groups at all - a hydrocarbon starting compound will deposit an essentially functionally blank surface. The provision of functionalised patterns on a non-fouling surface can be achieved by writing on a surface which has first been uniformly plasma polymerised by an ethylene oxide type monomer. Currently disclosed methods for patterning of plasma polymers do not allow the production of surfaces containing more than one chemistry. In addition, using this method of writing polymer onto a substrate permits formation on surface of closed loops and circuits - an aspect of surface patterning precluded by use of an overlayed mask.

Alternatively or in combination therewith, by manipulation of the plasma during the process, a pattern may be written that has along its length a variable concentration of chemistry (such as polymer or plasma), which is herein referred to as a "gradient surface", typically a microgradient. The invention encompasses a plasma polymerised surface that has along at least one axis (KYZ), typically along its length, a variable concentration of plasma or polymer. Further, the invention encompasses surfaces comprising multiple plasma polymers deposited in a controlled manner.

To form a gradient of chemistry, the composition of the plasma is changed concomitantly with the relative movement of the writing element with respect to the surface. Such a change in the composition of the plasma may be achieved by changing the temperature of the monomer(s), increasing the partial pressure or mixing ratio of the monomer(s) or carrier gas(es), or by changing the amplitude, or pulse regime, or frequency of the power input into the system.

According to a yet further aspect of the invention there is provided an assay product according to the invention for use with an array printer.

According to a further aspect of the invention there is provided an assay product according to the invention for use with an array reader.

The invention will now be described by examples only and with reference to the following figures, materials and methods;
Figure 1 is a plasma polymerisation apparatus;
Figure 2 is a photograph image of water vapour condensing on a 500 micron dot of perfluorohexane plasma polymer;
Figure 3 is a photograph image of water vapour condensing on a 100micron wide line of acrylic acid plasma polymer which contains a 90 degree bend;
Figure 4 is a graph showing the elemental composition, determined by XPS, of a gradient of acrylic acid/allylamine over a distance of 11mm.

### Materials and Methods

The methodology of plasma polymerisation is disclosed in WO01/31339 and is incorporated by reference in its entirety.

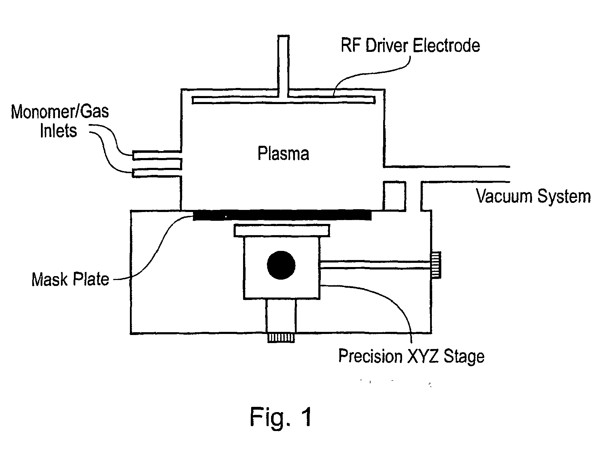

The schematic diagram of the plasma "writing" apparatus is shown in figure 1. The apparatus consists of two vacuum chambers separated by a Mask Plate, but sharing a common vacuum system. The topmost chamber has several monomer input ports and an electrode for exciting a plasma. The lower chamber contains a precision XYZ manipulation stage, upon which is mounted the substrate to be patterned.

The 'writer' element consists of a 'nib' which contains a small feature which is used to 'write' chemistry onto the surface. Examples of such nibs include single holes, multiple holes, and single or multiple slots where the dimensions may range from 2 microns up to several centimetres, but more typically lie in the range 5-1000 microns. The nib may be an integral part of the plasma source in (for example) the case of a microcapilliary. In this case the term 'nib' refers to the aperture at the end of the capilliary.

A plasma is initiated of such a composition (of monomer or monomers, or monomer(s) in conjunction with carriers gas or gases) as would be required to deposit a uniform film of the desired composition as described in WO01/31339.

Typically a monomer consists of an organic compound which may be induced to exist in the gas phase either by heating, or spraying, or by the use of a carrier gas, or by its own vapour pressure at room temperature or below. Pressure within the plasma chamber is typically around 1x10⁻² mbar, and normally within the range 10⁻³ mbar - 1 mbar. Working pressures for plasma polymerisation are normally between 10⁻⁵ mbar and atmospheric pressure, or higher.

Other plasma systems, for example, microwave, pulsed rf, dc, , atmospheric, microdischarge, microcapillary, may be used and the means of adapting the above description to allow these plasma sources to be integrated will be clear to one skilled in the art.

The writing element is translated across the surface of a sample mounted on an XYZ manipulator. Either the sample, or the plasma source, or both may be moved relative to the other. Such movement may be controlled manually, or by the action of computer controlled motors to describe the desired feature shape onto the surface. The rate of movement may be easily calculated by knowing the dimensions of the writing element, the deposition rate of the plasma polymer, and the required thickness of the deposited film.

To form a gradient of chemistry, the composition of the plasma is changed concomitantly with the relative movement of the writing element with respect to the surface. Such a change in the composition of the plasma may be achieved by changing the temperature of the monomer(s), increasing the partial pressure or mixing ratio of the monomer(s) or carrier gas(es), or by changing the amplitude, or pulse regime, or frequency of the power input into the system. Other means of altering the plasma composition are known in the art.

The sample is raised so as to be extremely close to the Mask Plate (but without touching). The mask plate consists of a stainless steel plate, with a small aperture that defines the features to be deposited. The nature of the deposition is such that the plasma is guided by the aperture and forms a polymeric deposit on the surface beneath it. Note however, that this aperture is used almost as a 'pen' to write functionalised polymeric material onto the substrate, as opposed to a simple 'stencil' to form an image on the surface.

Both chambers are evacuated using a common vacuum system consisting of a turbomolecular pump backed by a two-stage rotary pump. The base pressure of the whole apparatus is ∼10⁻⁵ mbar.

A plasma is excited in the top chamber, by means of an rf generator (Coaxial Power Systems, UK), and by adjusting the flow rate of the monomer/monomers and the power and pulse regime of the plasma the desired plasma composition is selected.

### "Writing" of plasma polymers as microdots

Allylamine was obtained from Aldrich (UK) and subjected to several freeze-pump-thaw cycles to remove dissolved gases prior to use. Silicon wafer was used as a substrate and after being cleaned with isopropyl alcohol was attached to the XYZ stage using double-sided sticky tape. A mask consisting of ~100 micron holes was attached to the Mask Plate and the substrate was raised to within a few microns of the Mask Plate.

A monomer flow rate of ~5sccm was set in the top chamber using fine-control needle valves. Subsequently, a plasma was excited in the top chamber and sustained for around 30 seconds to provide microdots of allylamine plasma polymer on the area of the substrate immediately beneath the mask plate.

Additional dots of carboxylic acid chemical functionality are written alongside the amine dots by changing the monomer compound from allylamine to acrylic acid.

### "Writing" of Plasma Polymers as Microtracks

The method is identical to that described above for plasma microdots. To deposit microtracks, the plasma composition is kept the same, and the sample is moved beneath the mask plate, effectively using the plasma to 'write' the tracks and features onto the substrate.

### "Writing" of Plasma Gradients

A functionality gradient was deposited by using two different monomer compounds. Allylamine and acrylic acid were obtained from Aldrich (UK) and subjected to several freeze-pump-thaw cycles to remove dissolved gases. A mask consisting of a single ~100 micron hole was attached to the mask plate, and a piece of silicon wafer as substrate was raised as close as possible to the mask without touching (as described above). Initially, a plasma was excited using only the acrylic acid monomer feed. The mixture of monomer gases was then varied concomitantly with the linear movement of the sample beneath the mask. Hence the initial deposition was comprised wholly of acrylic acid plasma polymer, while later deposition consisted of a mixture of allylamine and acrylic acid, and the final portion of the deposition consisted wholly of allylamine. Thus over the range of motion of the sample during the experiment, the surface composition changed smoothly from one dominated by carboxylic acid groups, to one in which amine groups dominated.

Microgradients are not simply limited to bi-functional gradients, any number of monomers could be used to produce continuously varying surface features. Similarly, gradients of other properties can be envisaged; gradients of wettability (from ultra-hydrophobic through to hydrophilic), gradients of crosslink density, adhesivity and variations of thickness. A gradient can be formed which comprises a chemically continuous region connecting any two or more polymers with different properties, irrespective of what those properties might be. A polymer can be seen as occupying a point in an n-dimensional parameter-space - there will always be a direct path between two such points, which is independent of the dimensionality of the parameter space.

The examples described above use a feature scale of around 100 microns, to illustrate the techniques. In practice it might be required that surfaces are patterned on a millimetre or centimetre scale as an upper boundary, right down to 1 micron at the bottom end.

There are variations that could be made to the plasma system to control the plasma writing process. Plasma may be excited using DC, radiofrequency (pulsed or continuous wave) or microwave radiation, or it may be excited within, or at the tip of a micrometre scale capillary. There may be carrier gases involved for some less-volatile monomers. The processes may benefit from a simple computer system to manage the plasma parameters and position of the XYZ stage for improved accuracy and automation of the writing process. Further, although the experiments described the plasma as being in a top chamber, and the sample in a lower chamber, the pattern formation requires only that the sample be isolated from the plasma by the mask plate, irrespective of orientation of the components of the system.

It is possible to change the plasma composition in the region of the mask by means of applied electric and magnetic fields. These might be used as 'lenses' to further focus the plasma. They may also be used to increase or decrease the relative contributions of the ionic and radical components of the plasma - in extremis reducing the species arriving at the substrate to a collimated beam of radical species, or low energy beam of ions.

In addition to directly depositing onto the substrate material, pretreatments may be employed to clean the surface, or to etch topographic features into the substrate prior to writing. This allows the construction of 3-dimensional functionalised structures on surfaces (for example a 'trench' with amine functional groups deposited along the bottom) in a single process.

There are a number of areas in which these plasma deposited patterns might be used. Microdots and microarrays may be used as microscopic 'test tubes' for chemical or biochemical interactions, for example in genomics and rapid screening of DNA, proteomics, and immunodiagnostics. The deposited functional groups may be used to immobilise entities such as DNA, RNA, proteins, peptides, polypeptides, ligands, proteoglycans, carbohydrates, nucleotides, oligonucleotides. Alternatively, they may act as reaction sites for subsequent derivatisation by chemical means.

The next level up from microdots and microarrays is to generate micropatterns of single functionalities. Stripes, tracks and more complex shapes may be deposited using different functional groups on the same substrate, allowing functional patterns containing different chemistries to exist on the same substrate and also allowing the formation of loops and circuits. These features might be used in microfluidics for transport of tiny volumes of liquid, as 'microvalves', adsorption of tiny quantities of reagent and to control adhesion properties.

Microgradients could be used to separate mixtures of biomolecules on the basis of difference in physical or chemical properties. (for example, mass, charge, size, hydrophobicity). This is analogous to gel electrophoresis, and gel permeation chromatography. Gradients could be used to separate out identical mixtures by different properties (charge, size, etc.).

The chemistry of the written features may range from non-functional hydrocarbon surfaces (deposited from alkane, alkene, aromatic type compounds) to any other conceivable chemical group. For example, amines, acids, alcohols, ethers, esters, imines, amides, keytones, aldehydes, anhydrides, halogens, thiols, carbonyls, silicones, fluorocarbons. Additionally, plasma polymers which are electrically conducting may be deposited. The only limitation on the functionality incorporated is that there must exist a starting compound that is capable of being induced to exist in the gas phase (with or without heating) at low pressure (above ~10⁻⁵ mbar). Different chemistries may also be formed using reactive (N₂, O₂, H₂O) or non-reactive (Ar) gases. These gases may also be used to etch features into the substrate - all as part of the same process.

Patterns may be produced that contain a mixture of any number of the above functionalities in any combination or arrangement on the same substrate material.

Surfaces that contain gradients of functionality on a scale of centimetres, down to around 10 microns are possible. A gradient is a region of continuous change between two different chemistries. A gradient can always be constructed between any two regions of different chemistry, in the same way that a straight line can always be drawn through two points in space.

The polymer micropattems, microarrays, microgradients and microtracks may be written onto any substrate material. For example, glasses, ceramics, metals, semiconductors, and polymers including (but not limited to) polycarbonate (PC), polystyrene (PS), polyethyleneterephthalate (PET), polymethylmethacrylate (PMMA), polyvinylchloride (PVC), polytetrafluoroethylene (PTFE).

### EXAMPLES

### Example 1

Perflurohexane was obtained from Aldrich (UK) and used as received, save for several freeze-pump-thaw cycles to remove dissolved gases prior to use. A single 13mm glass coverslip was used as a substrate, and cleaned with acetone and isopropyl alcohol before being placed on the XYZ manipulator and pumped down to the reactor base pressure (<10⁻³mbar). A 500 micron circular cross section writing element was placed between the excitation chamber, and the sample surface. A flow rate of perfluorohexane vapour of 2.4cm³ₛₜₚmin⁻¹ was achieved by using a fine-control needle valve. This gave a reactor pressure of 3.4x10⁻² mbar during deposition. A plasma was excited at a continuous wave power of 10W for a period of two minutes with the writing element remaining stationary throughout the deposition to leave a 'dot' of perfluorohexane chemistry.

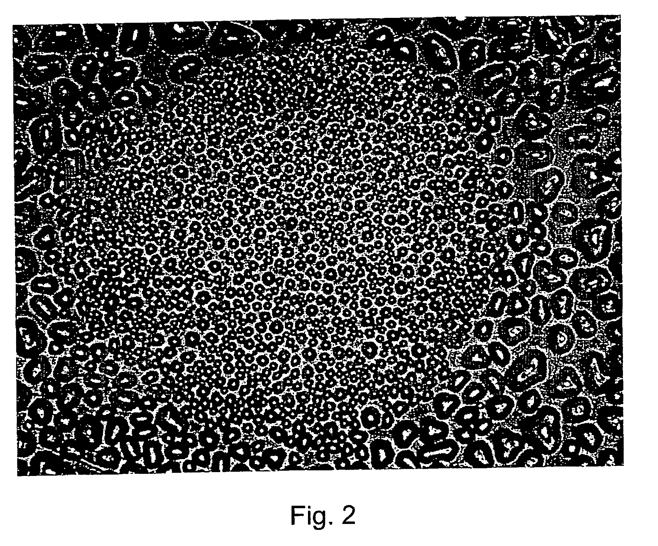

Fluorocarbon plasma polymers are by their nature hydrophobic, while the glass substrate is relatively hydrophilic. The dot was photographed using a conventional light microscope, with condensed water vapour showing areas of contrasting contact angle. This is shown in Figure 2. The different wettability of the plasma polymer compared to the background material causes the water droplets to have different shapes.

### Example 2

Acrylic acid and octa-1,7-diene monomers were obtained from Aldrich (UK) and used as received save for several freeze-pump-thaw cycles to remove dissolved gases prior to use. Initially, a homogenous layer of octadiene plasma polymer was deposited onto a single 13mm glass coverslip using a flow rate of 2cm³ₛₜₚmin⁻¹ and 10W continuous wave power to provide a hydrophobic background surface upon which to write hydrophilic (carboxylic acid) chemistry. The octadiene coated glass coverslip was placed on the XYZ manipulator and the system was pumped down to the reactor base pressure (<10⁻³mbar). A 100 micron writing element was placed between the excitation chamber and the sample surface. A flow rate of acrylic acid of 4cm³ ₛₜₚmin⁻¹ was set using a fine control needle valve (this gives a reactor pressure of 2.2x10⁻² mbar). A plasma was then excited at a pressure of 1.8x10-2 mbar and continuous wave power of 5W for a period of 2 minutes, during which time the writing element was translated across the surface first in the X-direction (at 0.5mm/min) for 1 min, then in the Y-direction (at 0.5mm/min) for 1 min.

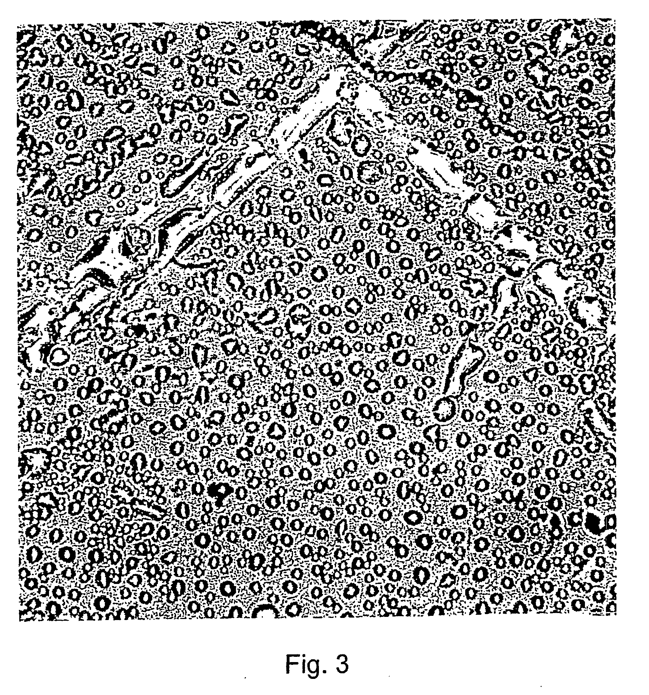

The deposited acrylic acid chemistry is much more hydrophilic than the background octadiene surface, hence the contrast between these two areas was visualised by condensing water vapour onto the surfaces, then viewing them directly using a conventional light microscope (Figure 3). Figure 3 shows a pair of straight lines at right angles written by the writing element. The image was formed by the same method described above.

### Example 3

Acrylic acid and allylamine monomers were obtained from Aldrich (UK) and used as received, save for several freeze-pump-thaw cycles to remove dissolved gases prior to use. A 13 mm glass coverslip was used as a substrate material and was attached to the XYZ sample stage and pumped down to the system base pressure (<10⁻³mbar). A 1 cm writing element was used and was initially placed so that the whole sample surface was exposed to the plasma. A plasma consisting of 4cm³ ₛₜₚmin⁻¹ of allylamine was excited in the plasma chamber at a continuous wave power of 5W and a reactor pressure of 1.9x10⁻² mbar. The writing element was moved across the surface at a rate of 1mm/min for a period of 13 minutes. Simultaneously, the flow rate of allylamine was reduced by slowly adjusting the needle valve, and replaced by a flow of acrylic acid vapour such that after 12 minutes, the monomer flow consisted of only acrylic acid at a flowrate of 4cm³ ₛₜₚmin⁻¹ and a pressure of ~2x10⁻² mbar. At all times the total monomer flow rate was maintained at 4 cm3stpmin-1. (The ratio of the two monomer flow rates in cm³ ₛₜₚmin⁻¹is equivalent to their molar ratio assuming ideal behaviour).

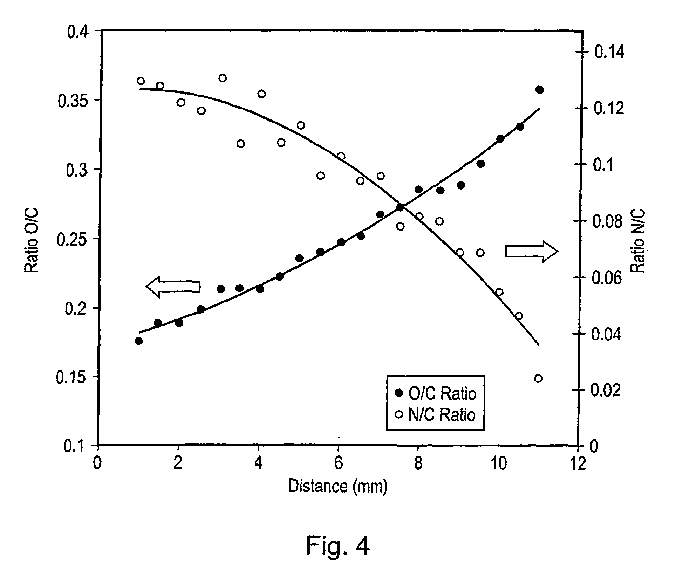

In order to analyse the gradual change of chemistry along the sample surface, it was analysed using X-Ray photoelectron spectroscopy at 500 micron intervals across the cover slip. The elemental composition at each point is shown in Figure 4 as a ratio of oxygen/carbon and nitrogen/carbon.

Figure 4 shows a gradient of oxygen and nitrogen chemistry which was changed concomitantly with the motion of the writing element from a composition of 100% allylamine to 100% acrylic acid at a constant power of 5W and a movement rate of the sample relative to the writing element of 1.0mm/min.

## Claims

1. A method to prepare at least part of at least one surface of a substrate comprising; depositing on said surface at least one plasma monomer wherein during deposition of said monomer, means are provided which move the monomer source across a surface to be treated to manufacture a non-uniform plasma polymer surface.

2. A method as claimed in claim 1 wherein said means moves said substrate relative to said monomer source.

3. A method as claimed in claim 1 wherein said means moves said monomer source relative to said substrate.

4. A method as claimed in any of claims 1 to 3 wherein the surface comprises at least one plasma polymer of at least one monomer wherein the concentration of said plasma polymer is non-uniform across said surface, or part thereof.

5. A method as claimed in any preceding claim wherein there is provided a surface comprising two or more plasma polymers formed from at least two monomers.

6. A method as claimed in any of claims 5 wherein the concentration of at least one plasma polymer is non-uniform across said surface, or part thereof.

7. A method as claimed in any preceding claim wherein the monomer is a volatile alcohol.

8. A method as claimed in any of claims 1 to 6 wherein said monomer is a volatile acid.

9. A method as claimed in any of claims 1 to 6 wherein the monomer is a volatile amine.

10. A method as claimed in any of claims 1 to 6 wherein the monomer is a volatile hydrocarbon.

11. A method as claimed in any of claims 1 to 6 wherein the monomer is a volatile fluorocarbon.

12. A method as claimed in any of claims 1 to 6 wherein the monomer is an ethyleneoxide-type molecule.

13. A method as claimed in any of claims 1 to 6 wherein the monomer is a volatile siloxane.

14. A method as claimed in any of claims 1 to 6 wherein said monomer is at least one selected from the group consisting of: allyl alcohol, acrylic acid, octa-1,7,-diene, allyl amine, perfluorohexane, tetraethyleneglycol monoallyl ether or hexamethyldisiloxane (HMDSO).

15. A method as claimed in any of claims 4 to 14 wherein said polymer consists of a single monomer.

16. A method as claimed in claim 15 wherein said monomer consists essentially of an ethylenically unsaturated organic compound.

17. A method as claimed in claim 16 wherein the monomer consists essentially of a single ethylenically unsaturated organic compound.

18. A method as claimed in claim 17 wherein the monomer consists of an ethylene oxide type molecule.

19. A method as claimed in claim 16 or 17 wherein the compound is an alkene, a carboxylic acid, an alcohol or an amine.

20. A method as claimed in claim 15 wherein the monomer consists of a mixture of two or more ethylenically unsaturated organic compounds.

21. A method as claimed in claim 20 wherein the compounds are selected from the group consisting of: an alkene, a carboxylic acid, an alcohol, or an amine.

22. A method as claimed in claim 15 wherein the monomer consists essentially of a saturated organic compound.

23. A method as claimed in claim 15 wherein the monomer consists essentially of an aromatic compound or a heterocyclic compound.

24. A method as claimed in any preceding claim wherein the monomer has a vapour pressure of at least 6.6x10⁻² mbar.

25. A method as claimed in any of claims 4 to 14 wherein the polymer is a co-polymer.

26. A method as claimed in claim 25 wherein the co-polymer comprises at least one organic monomer with at least one hydrocarbon.

27. A method as claimed in claim 26 wherein the hydrocarbon is an alkene.

28. A method as claimed in any preceding claim wherein the monomer (s) is/are deposited on said surface in spatially separated dots.

29. A method as claimed in any of claims 1 to 27 wherein the monomer (s) is/are deposited on said surface in tracks or lines.

30. A method as claimed in claim 28 or 29 wherein the dots and/or lines are of different polymer chemistry.

31. A method as claimed in claim 30 wherein the chemical composition of the line, track or dot is non-uniform along its length and in height.

32. A substrate comprising a surface obtainable by the method claimed in any of claims 1 to 31.

33. A substrate as claimed in claim 32 selected from the group consisting of: glass, plastics , nitrocellulose, nylon, metal, ceramics, quartz, metal films or silicon wafer.

34. An assay product comprising the substrate of claim 32 or 33.

35. A microassay comprising an assay product as claimed in claim 34.

36. A microtitre plate comprising an assay product as claimed in claim 35.

37. A substrate as claimed in claim 32 or 33 for use in separating cells and/or proteins and/or macromolecules.

38. A substrate as claimed in claim 32 or 33 further comprising a microfluidic device, or a part thereof.

## Patentansprüche

1. Verfahren zum Praparieren wenigstens Teil wenigstens einer Oberfläche eines Substrats, umfassend: Abscheiden auf dieser Oberfläche wenigstens eines Plasmamonomers, wobei bei der Abscheidung dieses Monomers Mittel bereitgestellt werden, die die Monomerquelle über eine zu behandelnde Oberfläche bewegen, um eine uneinheitliche Plasmapolymeroberfläche herzustellen.

2. Verfahren nach Anspruch 1, wobei das Mittel das Substrat relativ zur Monomerquelle bewegt.

3. Verfahren nach Anspruch 1, wobei das Mittel die Monomerquelle relativ zum Substrat bewegt.

4. Verfahren nach irgendeinem der Ansprüche 1 bis 3, wobei die Oberfläche wenigstens ein Plasmapolymer aus wenigstens einem Monomer umfasst, wobei die Konzentration des Plasmapolymers über die Oberfläche oder den Teil davon uneinheitlich ist.

5. Verfahren nach irgendeinem vorhergehenden Anspruch, wobei eine Oberfläche bereitgestellt wird, die zwei oder mehr Plasmapolymere umfasst, die aus wenigstens zwei Monomeren gebildet sind.

6. Verfahren nach Anspruch 5, wobei die Konzentration von wenigstens einem Plasmapolymer über die Oberfläche oder den Teil davon uneinheitlich ist.

7. Verfahren nach irgendeinem vorhergehenden Anspruch, wobei das Monomer ein flüchtiger Alkohol ist.

8. Verfahren nach irgendeinem der Ansprüche 1 bis 6, wobei das Monomer eine flüchtige Säure ist.

9. Verfahren nach irgendeinem der Ansprüche 1 bis 6, wobei das Monomer ein flüchtiges Amin ist.

10. Verfahren nach irgendeinem der Ansprüche 1 bis 6, wobei das Monomer ein flüchtiger Kohlenwasserstoff ist.

11. Verfahren nach irgendeinem der Ansprüche 1 bis 6, wobei das Monomer ein flüchtiger Fluorkohlenstoff ist.

12. Verfahren nach irgendeinem der Ansprüche 1 bis 6, wobei das Monomer ein Molekül vom Typ eines Ethylenoxids ist.

13. Verfahren nach irgendeinem der Ansprüche 1 bis 6, wobei das Monomer ein flüchtiges Siloxan ist.

14. Verfahren nach irgendeinem der Ansprüche 1 bis 6, wobei das Monomer wenigstens eines ist, das ausgewählt ist aus der Gruppe bestehend aus: Allylalkohol, Acrylsäure, Octa-1,7-dien, Allylamin, Perfluorhexan, Tetraethylenglycolmonoallylether oder Hexamethyldisiloxan (HMDSO).

15. Verfahren nach irgendeinem der Ansprüche 4 bis 14, wobei das Polymer aus einem einzigen Monomer besteht.

16. Verfahren nach Anspruch 15, wobei das Monomer im Wesentlichen aus einer ethylenisch ungesättigten organischen Verbindung besteht.

17. Verfahren nach Anspruch 16, wobei das Monomer im Wesentlichen aus einer einzigen ethylenisch ungesättigten organischen Verbindung besteht.

18. Verfahren nach Anspruch 17, wobei das Monomer aus einem Molekül vom Typ eines Ethylenoxids besteht.

19. Verfahren nach Anspruch 16 oder 17, wobei die Verbindung ein Alken, eine Carbonsäure, ein Alkohol oder ein Amin ist.

20. Verfahren nach Anspruch 15, wobei das Monomer aus einer Mischung aus zwei oder mehr ethylenisch ungesättigten organischen Verbindungen besteht.

21. Verfahren nach Anspruch 20, wobei die Verbindungen ausgewählt sind aus der Gruppe bestehend aus: einem Alken, einer Carbonsäure, einem Alkohol oder einem Amin.

22. Verfahren nach Anspruch 15, wobei das Monomer im Wesentlichen aus einer gesättigten organischen Verbindung besteht.

23. Verfahren nach Anspruch 15, wobei das Monomer im Wesentlichen aus einer aromatischen Verbindung oder einer heterocyclischen Verbindung besteht.

24. Verfahren nach irgendeinem vorhergehenden Anspruch, wobei das Monomer einen Dampfdruck von wenigstens 6,6 x 10⁻² mbar besitzt.

25. Verfahren nach irgendeinem der Ansprüche 4 bis 14, wobei das Polymer ein Copolymer ist.

26. Verfahren nach Anspruch 25, wobei das Copolymer wenigstens ein organisches Monomer mit wenigstens einem Kohlenwasserstoff umfasst.

27. Verfahren nach Anspruch 26, wobei der Kohlenwasserstoff ein Alken ist.

28. Verfahren nach irgendeinem vorhergehenden Anspruch, wobei das/die Monomer(e) auf der Oberfläche in räumlich getrennten Punkten abgeschieden wird/werden.

29. Verfahren nach irgendeinem der Ansprüche 1 bis 27, wobei das/die Monomer(e) auf der Oberfläche in Bahnen oder Linien abgeschieden wird/werden.

30. Verfahren nach Anspruch 28 oder 29, wobei die Punkte und/oder Linien polymerchemisch unterschiedlich sind.

31. Verfahren nach Anspruch 30, wobei die chemische Zusammensetzung der Linie, der Bahn oder des Punkts über deren Länge und Höhe uneinheitlich ist.

32. Substrat, umfassend eine Oberfläche, die nach dem Verfahren gemäß den Ansprüchen 1 bis 31 erhältlich ist.

33. Substrat nach Anspruch 32, ausgewählt aus der Gruppe bestehend aus: Glas, Kunststoff, Nitrocellulose, Nylon, Metall, Keramik, Quarz, Metallfolien oder Siliciumwafern.

34. Produkt für einen Assay, umfassend das Substrat nach Anspruch 32 oder 33.

35. Mikroassay, umfassend ein Assay-Produkt nach Anspruch 34.

36. Mikrotiterplatte, umfassend ein Assay-Produkt nach Anspruch 35.

37. Substrat nach Anspruch 32 oder 33 zur Verwendung beim Trennen von Zellen und/oder Proteinen und/oder Makromolekülen.

38. Substrat nach Anspruch 32 oder 33, ferner umfassend eine Mikrofluid-Vorrichtung oder einen Teil davon.

## Revendications

1. Procédé pour préparer au moins une partie d'au moins une surface d'un substrat comprenant ; déposer sur ladite surface au moins un monomère plasma, **caractérisé en ce que** durant le dépôt dudit monomère, des moyens sont prévus pour déplacer la source de monomère au travers d'une surface destinée à être traitée pour réaliser une surface polymère plasma non uniforme.

2. Procédé selon la revendication 1, **caractérisé en ce que** lesdits moyens déplacent ledit substrat par rapport à ladite source monomère.

3. Procédé selon la revendication 1, **caractérisé en ce que** lesdits moyens déplacent ladite source de monomère par rapport audit substrat.

4. Procédé selon l'une des revendications 1 à 3, **caractérisé en ce que** la surface comporte au moins un polymère plasma d'au moins un monomère, et **en ce que** la concentration dudit plasma monomère est non uniforme au travers de ladite surface, ou partie de celle-ci.

5. Procédé selon l'une des revendications précédentes, **caractérisé en ce qu'**il est prévu une surface comportant deux ou plusieurs polymères plasma formés à partir d'au moins deux monomères.

6. Procédé selon la revendication 5, **caractérisé en ce que** la concentration d'au moins un polymère plasma est non uniforme au travers de ladite surface ou partie de celle-ci.

7. Procédé selon l'une des revendications précédentes, **caractérisé en ce que** le monomère est un alcool volatile.

8. Procédé selon l'une des revendications 1 à 6, **caractérisé en ce que** ledit monomère est un acide volatile.

9. Procédé selon l'une des revendications 1 à 6, **caractérisé en ce que** le monomère est une amine volatile.

10. Procédé selon l'une des revendications 1 à 6, **caractérisé en ce que** le monomère est un hydrocarbone volatile.

11. Procédé selon l'une des revendications 1 à 6, **caractérisé en ce que** le monomère est un fluorocarbone volatile.

12. Procédé selon l'une des revendications 1 à 6, **caractérisé en ce que** le monomère est une molécule du type éthylèneoxyde.

13. Procédé selon l'une des revendications 1 à 6, **caractérisé en ce que** le monomère est un siloxane volatile.

14. Procédé selon l'une des revendications 1 à 6, **caractérisé en ce que** ledit monomère est au moins un choisi parmi le groupe consistant en : alcool allyle, acide acrylique, octa-1,7, -diène, amine allyle, perfluorohexane, tétrathylèneglycole, monallyle éther ou héxaméthyldisiloxane (HMDSO).

15. Procédé selon l'une des revendications 4 à 14, **caractérisé en ce que** ledit polymère consiste en un monomère unique.

16. Procédé selon la revendication 15, **caractérisé en ce que** le monomère consiste essentiellement en un composé organique insaturé en éthylène.

17. Procédé selon la revendication 16, **caractérisé en ce que** le monomère consiste essentiellement en un composé organique insaturé en éthylène, unique.

18. Procédé selon la revendication 17, **caractérisé en ce que** le monomère consiste en une molécule du type oxyde éthylène.

19. Procédé selon l'une des revendications 16 ou 17, **caractérisé en ce que** le composé est un alkène, un acide carboxylique, un alcool ou un amine.

20. Procédé selon la revendication 15, **caractérisé en ce que** le monomère consiste en un mélange de deux ou plusieurs composés organiques insaturés en éthylène.

21. Procédé selon la revendication 20, **caractérisé en ce que** les composés sont choisis parmi le groupe consistant en : un alkène, un acide carboxylique, un alcool ou un amine.

22. Procédé selon la revendication 15, **caractérisé en ce que** le monomère consiste essentiellement en un composé organique saturé.

23. Procédé selon la revendication 15, **caractérisé en ce que** le monomère consiste essentiellement en un composé aromatique ou un composé hétérocyclique.

24. Procédé selon l'une des revendications précédentes, **caractérisé en ce que** le monomère présente une pression de vapeur ou d'au moins 6,6x10⁻² mbar.

25. Procédé selon l'une des revendications 4 à 14, **caractérisé en ce que** le polymère est un copolymère.

26. Procédé selon la revendication 25, **caractérisé en ce que** le copolymère comporte au moins un monomère organique avec au moins un hydrocarbone.

27. Procédé selon la revendication 26, **caractérisé en ce que** l'hydrocarbone est un aikène.

28. Procédé selon l'une des revendications précédentes, **caractérisé en ce que** le ou les monomères est ou sont déposé(s) sur ladite surface en des points séparés de manière spatiale.

29. Procédé selon l'une des revendications 27, **caractérisé en ce que** le ou les monomère(s) est ou sont déposé(s) sur ladite surface en ligne ou piste.

30. Procédé selon l'une des revendications 28 ou 29, **caractérisé en ce que** les points et ou lignes sont de différentes chimies polymères.

31. Procédé selon la revendication 30, **caractérisé en ce que** la composition chimique de la ligne ou piste ou point est non uniforme le long de sa longueur et en hauteur.

32. Substrat comprenant une surface susceptible d'être obtenue par le procédé selon l'une des revendications 1 à 31.

33. Substrat selon la revendication 32 choisi parmi le groupe consistant en : verre, plastique, nitrocellulose, nylon, métal, céramique, quartz, film métallique et tranche de silicium.

34. Produit d'analyse comprenant le substrat selon l'une des revendications 32 ou 33.

35. Microanalyse comprenant un produit d'analyse selon la revendication 34.

36. Plaque microtitre comprenant un produit d'analyse selon la revendication 35.

37. Substrat selon la revendication 32 ou 33, destiné à être utilisé pour la séparation de cellules et/ou de protéines et/ou de macromolécules.

38. Substrat selon l'une des revendications 32 ou 33, comprenant en outre un dispositif microfluide, ou une partie de celui-ci.
